# EUROPEAN PATENT APPLICATION

(11) **EP 1 288 221 A1**
(43) Date of publication of application: **05.03.2003**
(21) Application number: 01936844.8
(22) Date of filing: 04.06.2001
(51) Int. Cl.: C07K 7/06, A61K 38/08, A61P 17/14

(54) **OLIGOPEPTIDES**

(30) Priority: 05.06.2000 JP 2000166903
(71) Applicant: Sumitomo Electric Industries, Ltd., Osaka-shi, Osaka 541-0041 (JP)
(72) Inventor: OKA, Yumiko, Yokohama-shi, Kanagawa 244-8588 (JP); HIRAI, Yohei, Yokohama-shi, Kanagawa 244-8588 (JP); KOSHIDA, Shogo, Yokohama-shi, Kanagawa 244-8588 (JP); TAKEBE, Kyoko, Yokohama-shi, Kanagawa 244-8588 (JP); TSUDA, Hokari, Yokohama-shi, Kanagawa 244-8588 (JP)
(74) Representative: Sternagel, Fleischer, Godemeyer & Partner Patentanwälte
(86) International application number: JP0104691
(87) International publication number: WO01094382

(57) **Abstract**

An oligopeptide or a salt thereof represented by the following amino acid sequence (I):
Lys-Ser-Ile-Glu-Gln-Ser-Cys-Asp-Gln-Asp-Glu which is useful for an active ingredient of a medicament such as a hair growth promoting agent, or the above oligopeptide which includes amino acid variations, or the above oligopeptide modified with biotin and the like.

## Description

### Technical Field

This invention relates to an oligopeptide having hair growth promoting activity and a hair growth promoting agent comprising said oligopeptide as an active ingredient.

### Background Art

The normal morphogenesis of epithelial tissue has been suggested to be controlled by factors derived from mesenchymal cells present around the epithelial tissue. Diseases resulting from the abnormal morphogenesis of epithelial tissue are largely caused by abnormalities of mesenchymal cells. Therefore, an interest has arisen in the clarification of the mechanism in which mesenchymal cells controls the morphogenesis of epithelial tissue. However, substances involved in the control of morphogenesis of epithelial tissue are expressed under time and spatial control in a complicated system, and accordingly, it is extremely difficult to isolate these substances and analyze their functions. It is also difficult to construct a model experimental system simplifying the morphogenesis of epithelial tissue. For these reasons, no significant progress has been made to date in researches in this field. Thus, analysis of the controlling mechanism for the morphogenesis of epithelial tissue has been highly desired in order to elucidate the mechanism of occurrence of diseases associated with the morphogenesis of epithelial tissue and establish methods for treating these diseases.

Under the circumstances, epimorphin involved in the control of the morphogenesis of epithelial tissue was isolated (Japanese Patent Laid-Open Publication No. 25295/94). This substance, being a physiologically active substance containing a protein consisting of 277 to 289 amino acids as a core protein, was revealed to be biosynthesized mainly by mesenchymal cells. It was also found that epimorphin had the action of promoting the morphogenesis of epithelial tissue through is action on epithelial cells, and that normal tissue formation was not progressed when epimorphin failed to function.

As to the structure of epimorphin, it has been found that epimorphin molecule can be roughly divided into four fragments from a structural viewpoint (EP 0698666). That is, the polypeptide consisting of full length of epimorphin can be divided into the following four fragments, from its N-terminal, a coiled coil domain (1), a functional domain (2), a coiled coil domain (3), and hydrophobic domain at the C-terminal. In these fragments, it is suggested that the functional domain (the domain specified by 104th to 187th amino acids in human epimorphin) participates in cell adhesion and is closely related with an expression of physiological activity of epimorphin (the aforementioned EP publication).

Since epimorphin has an action for promoting normal morphogenesis, this substance is expected to be useful as an active ingredient of medicaments for preventive or therapeutic treatment of diseases caused by abnormal morphogenesis, or medicaments such as a hair growth promoting agent. However, a native epimorphin obtained from a mammal is almost insoluble in an aqueous media such as saline, which causes difficulty in practically using the substance as medicaments. Some attempts were made to produce new epimorphin derivatives having good solubility while substantially keeping the promoting activity on morphogenesis of the native epimorphin. For example, a modified form (fragment 123) obtained by removing a hydrophobic region at C-terminal has been known (Japanese Patent Laid-Open Publication No. 25295/1994).

It is also known that a polypeptide having a partial structure of epimorphin promotes morphogenesis of epithelial tissue through its action on epithelial cells (International Publication WO98/22505). This peptide is soluble in aqueous media such as saline, and the above publication teaches that this peptide has hair growth promoting activity. However, no biological activity of a shorter fragment as a partial structure of this peptide has been clarified.

### Disclosure of the Invention

An object of the present invention is to provide an oligopeptide having hair growth promoting activity. Another object of the present invention is to provide a medicament, preferably, a hair growth promoting agent comprising said oligopeptides as an active ingredient.

The inventors of the present invention conducted extensive studies to achieve the aforementioned objects, and as a result, they found that some oligopeptides having partial structures of epimorphin are excellent in hair growth promoting activity. The present invention was achieved on the basis of these findings.

The present invention thus provides an oligopeptide or a salt thereof selected from the group consisting of:
an oligopeptide of the following (1) and (2)
(1) an origopeptide represented by: 
amino acid sequence (I);
Lys-Ser-Ile-Glu-Gln-Ser-Cys-Asp-Gln-Asp-Glu
 
amino acid sequence (II);
Ser-Ile-Glu-Gln-Ser-Cys-Asp-Gln-Asp-Glu
 
amino acid sequence (III);
Ser-Ile-Glu-Gln-Ser-Cys-Asp-Gln-Asp
 
amino acid sequence (IV);
Ser-Ile-Glu-Gln-Ser-Cys-Asp-Gln
 
amino acid sequence (V);
Ser-Ile-Glu-Gln-Ser-Cys-Asp
 
amino acid sequence (VI);
Ser-Ile-Glu-Gln-Ser-Cys
 
amino acid sequence (VII);
Ile-Glu-Gln-Ser-Cys-Asp-Gln-Asp-Glu
 
amino acid sequence (VIII);
Glu-Gln-Ser-Cys-Asp-Gln-Asp-Glu, or
 
amino acid sequence (IX); or
Gln-Ser-Cys-Asp-Gln-Asp-Glu
(2) an oligopeptide having an amino acid sequence wherein one to several amino acids are deleted, replaced, or added in any one of the amino acid sequences (I) to (IX), and having substantially the same hair growth promoting activity as that of the oligopeptide represented by any one of the amino acid sequences (I) to (IX), and a modified oligopeptide based on the oligopeptide defined in (1) or (2), which has substantially the same hair growth promoting activity as that of the oligopeptide represented by any one of the amino acid sequences (I) to (IX).

According to a preferred embodiment of the above invention, there is provided an oligopeptide or a salt thereof represented by any one of the amino acid sequences (I) to (IX), or the modified oligopeptide thereof having substantially the same hair growth promoting activity as that of the oligopeptide represented by any one of the amino acid sequences (I) to (IX). A more preferred embodiment includes an oligopeptide or a salt thereof represented by any one of the amino acid sequences (II) to (V), or the modified oligopeptide thereof having substantially the same hair growth promoting activity as that of the oligopeptide represented by any one of the amino acid sequences (II) to (V). According to a more preferred embodiment of the above invention, there is provided an oligopeptide represented by any of the amino acid sequences (I) to (IX), and most preferably, an oligopeptide or salt thereof represented by any one of the amino acid sequence (I) or the amino acid sequence (IX) is provided.

An example of the modified oligopeptide includes biotinylated oligopeptide, and a more preferred example of the modified oligopeptide includes an oligopeptide of which N-terminal is bound by biotin with or without a spacer. Another example of the modified oligopeptide includes an oligopeptide dimerized by sulfhydryl group of cystein residue in the oligopeptide.

From another aspect, there is provided a medicament comprising the above oligopeptide or a physiologically acceptable salt thereof as an active ingredient. The medicament can be used as a hair growth promoting agent for a mammal. Further, there are provided a use of the above oligopeptide or a salt thereof for manufacture of the above medicament, preferably, a hair growth promoting agent; and a method for promoting hair growth, which comprises a step of administering an effective amount of the above oligopeptide or a physiologically acceptable salt thereof to a mammal including human.

### Brief Description of the Drawings

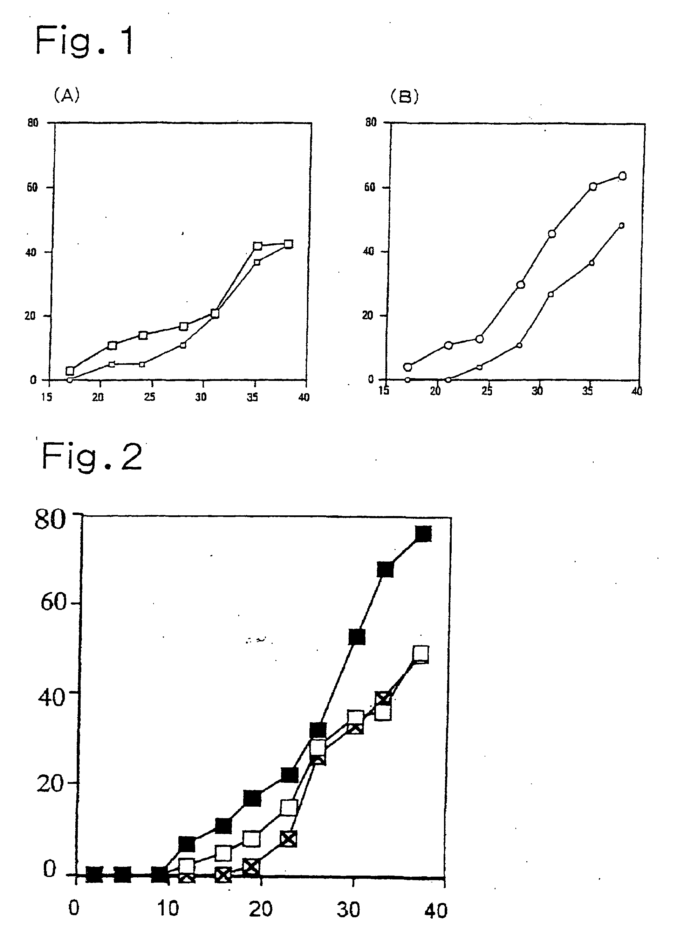

Fig.1 shows a hair growth promoting activity of the oligopeptide (represented by the amino acid sequence (II)) of the present invention. In the figure, (A) show the result obtained by a biotinylated oligopeptide. Square (large) shows the result obtained by oligopeptide b7, and square(small) shows the result obtained by the control. (B) shows the result obtained by a S-S bridged and biotinylated oligopeptide. Round (large) shows the result obtained by oligopeptide ssb7, and round (small) shows the result obtained by the control. The vertical axis indicates hair growth score, and the horizontal axis indicates the day from the start of application.
Fig.2 shows a hair growth promoting activity of the oligopeptide of the present invention (S-S bridged and biotinylated oligopeptide:ss7). In the figure, ■ indicates the of S-S bridged and biotinylated oligopeptide, □ indicates the result obtained from the first control (same as the control in Fig.1) , and 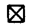 shows the result of the second control (random 7-mer oligopeptide). The vertical axis indicates hair growth score, and the horizontal axis indicates the day from the start of application.

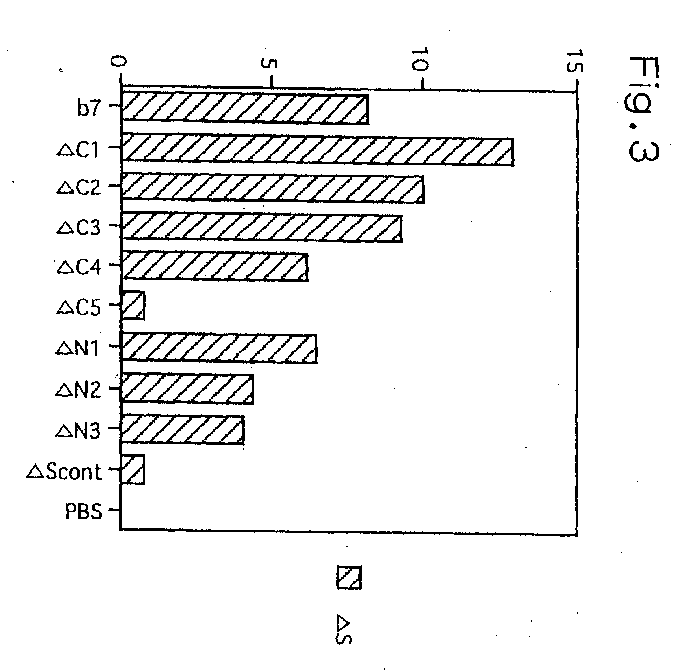

Fig.3 shows the result of evaluation of b7 Δ C1, b7 Δ C2, b7 Δ C3, b7 Δ C4, b7 Δ C5, b7 Δ N1, b7 Δ N2, and b7 Δ N3 on IL-8 inducing activity. In the figure, Scont indicates the result of blocking reagent, PBS indicates the result of phosphate buffered saline, and the vertical axis indicates the relative value of the secretion amount of IL-8.

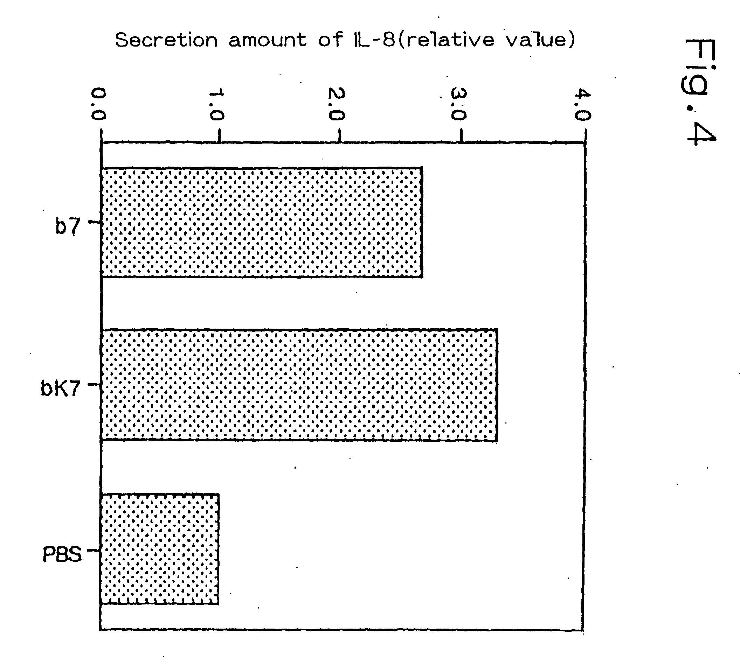

Fig.4 shows the results of evaluation of oligopeptide bk7 obtained by binding biotin to the N-terminal of the oligopeptide represented by the amino acid sequence (I) and oligopeptide b7 on IL-8 inducing activity. In the figure, PBS indicates the result of phosphate buffered saline, and the vertical axis indicates the relative value of the secretion amount of IL-8.

### Best Mode for Carrying out the Invention

The oligopeptide represented by the above amino acid sequence (I) of the present invention is an oligopeptide consisting of 11 amino acids residue at the C-terminal of the polypeptide (II) disclosed in International Publication WO98/22505. The oligopeptide represented by the above amino acid sequence (II) corresponds to an oligopeptide obtained by deleting one amino acid (Lys) from the N-terminal of the above amino acid sequence (I). The oligopeptides represented by the above amino acid sequences (III) to (VI) correspond to oligopeptides obtained by deleting one, two, three, and four amino acid residues from the C-terminal of the above amino acid sequence (II), respectively. The oligopeptides represented by the above amino acid sequences (VII) to (IX) correspond to oligopeptides obtained by deleting one, two, and three amino acids from the N-terminal of the above amino acid sequence (II), respectively. The oligopeptides represented by the above amino acid sequences (I) to (IV) have hair growth promoting activity as specifically shown in the following Examples. Although it is not intended to be bound by any specific theory, there is a possibility that the cystein of the C-terminal of the amino acid sequence (VI) is essential for the activity.

Oligopeptides having an amino acid sequence wherein one to several amino acids are deleted, replaced, or added in any one of the amino acid sequences (I) to (IX) having substantially the same hair growth promoting activity as that of the oligopeptide represented by any of the amino acid sequences (I) to (IX) (hereinafter occasionally, referred to as "variant oligopeptide") fall within the scope of the present invention. Further, modified oligopeptides based on the above oligopeptide represented by any one of the above amino acid sequences (I) to (IX) or the above variant oligopeptide, which have substantially the same hair growth promoting activity as that of the oligopeptide represented by any one of the amino acid sequences (I) to (IX) also fall within the scope of the present invention. One of ordinary skill in the art can easily ascertain that the above variant oligopeptide and modified oligopeptide have substantially the same hair growth promoting activity as that of the oligopeptide represented by any one of the amino acid sequences (I) to (IX) by the test method specifically described in detail in the Examples of the present specification or by adding alteration or modification to said test method.

In the above variant oligopeptide, the kind of amino acid to be added or replaced is not particularly limited. L-Amino acids are preferred. The number and position of the amino acid to be added or replaced is also not particularly limited, for example, the number of amino acid to be added is 1 to 5, preferably 1 to 3, more preferably 1 to 2. One to several amino acids, preferably 1 to 5, more preferably 1 to 3 amino acids may be added to the N-terminal or the C-terminal.

In the above modified oligopeptide, the term "modified" must be construed in the broadest sense including chemical modification and biological modification. Examples of the modification include introduction of a functional group such as alkylation, esterification, halogenation, and amination, or conversion of a functional group such as oxidation, reduction, addition, and elimination, or introduction of a sugar compound (a monosaccharide, disaccharide, oligosaccharide, or polysaccharide) or a lipid compound, phosphorylation, biotinylation. However, the modifications are not limited to these examples.

An example of the preferred modified oligopeptide includes a biotinylated oligopeptide, and a more preferred example includes an oligopeptide of which N-terminal is bound by biotin with or without a spacer. In the above modified oligopeptide, an appropriate chemical modification may be added to the biotin as long as the desirable physiological activity is maintained. A method for producing the biotinylated oligopeptide is specifically shown in the Examples of the present specification. In order to introduce biotin to the N-terminal by means of a spacer having an appropriate length, for example, NHS-Biotin or NHS-LC-Biotin (available from Pierce) can be used.

Another preferred example of the modified oligopeptide includes an oligopeptide dimerized by sulfhydryl group of cystein residue therein.

The above oligopeptides may be in free form, or may be provided as acid addition salts or base addition salts. Examples of the acid addition salts include mineral acid salts such as hydrochloride, sulfate, nitrate, and phosphate; organic acid salts such as para-toluenesulfonate, methanesulfonate, citrate, oxalate, maleate, and tartrate. Examples of the base addition salts include metal salts such as sodium salt, a potassium salt, a calcium salt, and a magnesium salt; an ammonium salt; organic ammonium salts such as a methyl ammonium salt, and a trimethyl ammonium salt. The oligopeptide may form a salt with amino acids such as glycine, or may form a zwitter ion in the molecule.

Any polymers of the oligopeptide such as a dimer fall within the scope of the present invention. For example, a dimerized oligopeptide by S-S bond falls within the scope of the present invention. Further, these oligopeptides may exist in a form of a hydrate or a solvate. The above oligopeptides have plural asymmetric carbon atoms. Although the stereochemistry of each asymmetric carbon atoms is not limited, it is preferable that the amino acid reside is L-amino acid. Stereoisomers such as optical isomers or diastereomers based on the asymmetric carbon atoms, any mixtures of the stereoisomers, and racemates fall within the scope of the present invention.

The oligopeptide of the present invention can be synthesized by a conventional chemical technique for peptide synthesis, such as solid phase or liquid phase method. There are various kinds of articles about protective group for amino group or the like and condensation agent for a condensation reaction in the filed of peptide synthesis, and accordingly, these articles can be referred to for the synthesis. In the solid phase method, commercially available various peptide synthesizers can be utilized. As for a method for introducing and removing a protective group, for example, Protective Groups in Organic Synthesis, T.W.Greene, John Wiley & Sons, Inc. 1981 and the like can be referred to.

By Applying an ordinary biological method such as a gene expression procedure, a desired oligopeptide can be obtained by constructing a recombinant vector containing a DNA sequence encoding the above oligopeptide, preparing a microorganism (transformant) transformed by the vector, and separating and purifying the oligopeptide from culture of the transformant. The method for producing the oligopeptides is not limited to these chemical and biological methods. Methods for producing modified oligopeptides including chemical modification and biological modification are well known to one of ordinary skill in the art, and any methods can be used.

The oligopeptide of the present invention is useful for an active ingredient of a medicament used as a hair growth promoting agent and the like. The term "medicament" is herein used in the broadest sense including a hair growth promoting agent which is sometimes classified into a quasi-drug, as well as a medicament for preventive or therapeutic treatment of diseases of a mammal including human. The term "hair growth promoting agent" used herein should be construed in the broadest sense including stimulation of hair generation and hair growth promotion, and prevention of hair loss, and the term should not be construed in any limiting sense.

As the medicament of the invention, one or more substances selected from among the aforementioned oligopeptides or their physiologically acceptable salt, per se, may be used. Generally, however, it is preferable to prepare and administer a pharmaceutical composition comprising one or more of the above substances as an active ingredient by using one or more pharmaceutically acceptable pharmaceutical additives. A hair growth promoting agent containing one or more of the aforementioned oligopeptides can be applied in a form of external preparations such as a cream, a spray, a coating solution, and a patch. The agent can be administered to a target site directly in a form of an injection. It is possible to provide the agent in any form suitable for the purpose of use as a hair growth promoting agent.

For example, the above oligopeptides as an active ingredient may be added to a shampoo or a rinse, or the above oligopeptide can be encapsulated into a liposome to manufacture a preparation. The composition in the aforementioned forms also falls within the medicament of the present invention. In order to achieve an effective transdermal absorption of the above oligopeptides through the keratin layer of skin, it is preferable to add an appropriate detergent, lipid-soluble substance of the likein a cream. The dose of the above hair growth promoting agent can be selected suitably depending on the purpose of application, the form of the agent, a kind of the active ingredient and the like. For example, it is possible to determine a dose by referring to the dose specifically shown in the Examples of the present specification.

### Examples

The present invention will be explained more specifically by the following Examples. However, the scope of the invention is not limited to these examples.

### Example 1

An oligopeptide having the above amino acid sequence (II) was synthesized by the solid phase method using Fmoc (sometimes referred to as "pep7" in the following examples). Also, a modified oligopeptide (sometimes referred to as "b7" in the following examples) with modification by biotin at the N-terminal. Each of the synthesized oligopeptides was purified by high-performance liquid chromatography (HPLC), and their purities were verified to be 90% or higher by HPLC and Mass. The conditions of HPLC were as follows:
Column: ODS-UG3 (Monomeric ODS, Nomura Kagaku), 1.0 mm in inside diameter, 100 mm in length
Measurement: room temperature (25°C)
Detection: UV 214 nm, 280 nm
Eluenting solvent: gradient of solvent A and solvent B (solvent A: 0.1% trifluoroacetic acid; solvent B: 90% acetonitrile/0.1% trifluoroacetic acid, linear concentration gradient from 5 minutes after (solvent B: 0%) to 55 minutes (solvent B: 55%)
Flow rate: 75 ml/ml
Retention time
pep7: 21.52 minutes (dimer), 20.59 minutes (monomer)
b7: 29.89 minutes (monomer), 32.85 minutes (dimer)

### Example 2

The oligopeptide prepared in Example 1 was dissolved in phosphate buffered saline (PBS) so as to give the concentration of 0.3 mg/ml, and this solution was added with the same volume of 100% ethanol to prepare 50% ethanol/PBS solution at the concentration of 0.15 mg/ml. The oligopeptide was crosslinked as follows. To the oligopeptide solution (5 ml) dissolved in PBS at the concentration of 1 mg/ml, BMH dissolved in dimethylsulfoxide (65 µl) was gradually added with stirring so as to give the concentration of 33 µg/µl, and the reaction was carried out at 4°C overnight. The solution was further added with PBS (6.6ml) and a solution of cysteine hydrochloride dissolved in PBS (5ml) so as to give the concentration of 5 mg/ml, and mixed to prepare S-S bridged oligopeptide solution at the final concentration of 0.3 mg/ml (hereinafter in the examples, the above oligopeptide obtained by crosslinking of pep7 is sometimes referred to as "ss7" and the biotinylated ss7 sometimes as "ssb7"). The retention time is 33.01 minutes by HPLC under the aforementioned conditions. A control solution was prepared by reacting in the same manner with the addition of the reagents except for using PBS instead of the oligopeptide solution. A solution of the crosslinked oligopeptide was also added with the same volume of ethanol to prepare a 50% ethanol/PBS solution at the final concentration of 0.15 mg/ml.

C3H and C57BL/6 mice are known to have sustained telogen for about 50 days from the 45th day after the birth to around the 95th day. Their hair cycle is easily judged based on the skin color changes, i.e., from pink in telogen to gray or black in anagen. A test for evaluating whether or not the administration the oligopeptides of the present invention promotes the transition from telogen to anagen was carried out using the mice. Seven weeks old (48 to 50-day old, female) C57BL/6 mice were purchased and hair of the back (about 3 × 2.5 cm²) was carefully shaved with electric clippers for animals so as not to injure the skin, and the hair cycle was confirmed to be in telogen from the skin color. The oligopeptide solution prepared above was applied to five mice in each group, once a day and 5 days in a week in the amount of 0.2 ml until 38th day from the start of the test. The application was carried out by using a syringe without needle. Dipetide (Ile-Lys) and tripeptide (Glu-Ile-Lys) of which N-terminal is biotinylated were mixed, and then added with the cross-linking agent to prepare a control solution.

Plural persons (two persons) observed the mice twice a week by naked eye for evaluation, and gave a 6-graded score depending on a ratio of hair restoration area based on the hair shaved area. At the same time, photographs were taken. Hair grow scores were calculated as follows. At first, the following score was given depending on a ratio of areas where the skin color changes to gray or black in the hair shaved area; 0-20% : 1, 20-40% : 2, 40-60% : 3, 60-80% :4, 80-100% : 5. The sum of the above scores in each group was determined as the hair growth score. The maximum value of the hair growth score of each group was 50 for each of the persons for judgment, and the maximum value of hair growth score was 100 because the judgment was made by two persons. In the group wherein the S-S bridged and biotynalated oligopeptide was applied, the transition to anagen was 7 days or more earlier than the control group, and the hair restoration was apparently promoted at any time until the hair restored and growed almost completely. On the other hand, in the group wherein the biotinylated oligopeptide was applied, the hair restoration was promoted until about 30 days from the start of the test as compared with the control group similarly to the S-S bridged group. The results are shown in Figure 1. The result of ss7 is shown in Figure 2. From these results, it is considered that the oligopeptide represented by the amino acid (II) has hair growth promoting activity.

### Example 3

Oligopeptide b7 Δ C1, b7 Δ C2, b7 Δ C3, b7 Δ C4, and b7 Δ C5 were synthesized by deleting one, two, three, four, or five amino acids from the C-terminal of b7, respectively, and then blocking sulfhydryl group. Oligopeptide b7 Δ N1, b7 Δ N2, and b7 Δ N3 were synthesized by deleting one, two, or three amino acids from the N-terminal of b7. Oligopeptide bk7 (without block of sulfhydryl group) represented by the amino acid sequence (I) was synthesized whose N-terminal was bound with lysine.

Human keratinocytes (NHEK cell, Clonetics, available from Sanko Jyun-yaku, Ltd.) were cultivated in a medium for proliferation (Clontics) comprising 30 µg/ml BPE, 0.1ng/ human EGF, 5 µg/ml insulin, 0.5 µg/ml hydrocortisone, 50 µg/ml gentamycin, and 50 ng/ml amphoterin. These cells were re-suspended at the concentration of 1 × 10⁴ cells/ml in a medium wherein EGF, insulin, and hydrocortisone were removed from the above medium for proliferation, and then 100 µl of the suspension was placed on each well of a 96-well plate. At the same time of the plating of the cells, 5 µl of 1 mg/ml oligopeptide was added to the suspension so as to be final concentration of 50 µg/ml. After cultivation for 16-20 hours, the amount of IL-8 in the culture supernatants was measured by ELISA kit (ENDOGEN). There was a good correlation between inducing activity on IL-8 secretion by NHEK cells and hair growth promoting activity (Table 1).

**Table 1**

| Oligopeptide | IL-8 inducing activity | Hair growth Activity |
|---|---|---|
| b7 | ⓞ | ○ |
| ssb7 | ⓞ | ⓞ |
| ss7 | ⓞ | ⓞ |
| control | × | × |

The results obtained by evaluation of oligopeptides b7 Δ C1, b7 Δ C2, b7 Δ C3, b7 Δ C4, b7 Δ C5, b7 Δ N1, b7 Δ N2, and b7 Δ N3 on IL-8 inducing activity are shown in Figure 3. When one or more amino acids were deleted from the N-terminal, the secretion amount of IL-8 was slightly decreased, whilstwhen the one or more amino acids were deleted from the C-terminal, the secretion amount of IL-8 was maintained until deletion of four amino acids. When five amino acids were deleted, the secretion amount of IL-8 was significantly decreased. From these results, it is revealed that almost the same hair growth activity as that of oligopeptide b7 can be expected by the deletion of up to four amino acids are deleted from the C-terminal, or the deletion of up to three amino acids from the N-terminal. As shown in Figure 4, 11-mer oligopeptide bk7 had also almost the same IL-8 inducing activity as that of oligopeptide b7.

### Example 4

The N-terminal of oligopeptide pep7 was biotinylated using NHS-biotin or NHC-biotin (Pierce) in accordance with the instructions of the attached manual. When NHS-biotin was used, -O-CO-(CH₂)₄-(13.5Å) was introduced as a spacer between the N-terminal and the biotin, and when NHS-LC-biotin was used, -O-CO-(CH₂)₅-NH-CO-(CH₂)₄-(22.4Å) was introduced as a spacer between the N-terminal and the biotin. The secretion amount of IL-8 was determined using these biotinylated oligopeptides in the same manner as in Example 3. These oligopeptides had almost the same IL-8 inducing activity as that of oligopeptide b7. These results show that the oligopeptide of which N-terminal is directly biotinylated has almost the same hair growth promoting activity as that of the oligopeptide of which N-terminal is biotinylated by means of a spacer, and both of them are activated as compared with the oligopeptide without being introduced with biotin.

### Industrial Applicability

The oligopeptide of the present invention has hair growth promoting activity and are useful as an active ingredient of a medicament such as a hair growth promoting agent.

## Claims

1. An oligopeptide or a salt thereof selected from the group consisting of:
an oligopeptide of the following (1) and (2)
(1) an origopeptide represented by: 
amino acid sequence (I);
Lys-Ser-Ile-Glu-Gln-Ser-Cys-Asp-Gln-Asp-Glu
 
amino acid sequence (II);
Ser-Ile-Glu-Gln-Ser-Cys-Asp-Gln-Asp-Glu
 
amino acid sequence (III);
Ser-Ile-Glu-Gln-Ser-Cys-Asp-Gln-Asp
 
amino acid sequence (IV);
Ser-Ile-Glu-Gln-Ser-Cys-Asp-Gln
 
amino acid sequence (V);
Ser-Ile-Glu-Gln-Ser-Cys-Asp
 
amino acid sequence (VI);
Ser-Ile-Glu-Gln-Ser-Cys
 
amino acid sequence (VII);
Ile-Glu-Gln-Ser-Cys-Asp-Gln-Asp-Glu
 
amino acid sequence (VIII);
Glu-Gln-Ser-Cys-Asp-Gln-Asp-Glu, or
 
amino acid sequence (IX); or
Gln-Ser-Cys-Asp-Gln-Asp-Glu
(2) an oligopeptide having an amino acid sequence wherein one to several amino acids are deleted, replaced, or added in any one of the amino acid sequences (I) to (IX), and having substantially the same hair growth promoting activity as that of the oligopeptide represented by any one of the amino acid sequences (I) to (IX), and a modified oligopeptide based on the oligopeptide defined in (1) or (2), which has substantially the same hair growth promoting activity as that of the oligopeptide represented by any one of the amino acid sequences (I) to (IX).

2. The oligopeptide or a salt thereof according to claim 1, wherein the modified oligopeptide is a biotinylated oligopeptide.

3. The oligopeptide or a salt thereof according to claim 2, wherein the modified oligopeptide is an oligopeptide of which N-terminal is bound by biotin with or without a spacer.

4. The oligopeptide or a salt thereof according to claim 1, wherein the modified oligopeptide is an oligopeptide dimerized by sulfhydryl group of cystein residue in the amino acid sequences described above.

5. An oligopeptide or a salt thereof represented by any one of the amino acid sequences (I) to (IX) described in claim 1, and a modified oligopeptide thereof, which has substantially the same hair growth promoting activity as that of the oligopeptide represented by any one of the amino acid sequences (I) to (IX).

6. The oligopeptide or a salt thereof according to claim 5, wherein the modified oligopeptide is a biotinylated oligopeptide.

7. The oligopeptide or a salt thereof according to claim 6, wherein the modified oligopeptide is an oligopeptide of which N-terminal is bound by biotin with or without a spacer.

8. The oligopeptide or a salt thereof according to claim 5, wherein the modified oligopeptide is an oligopeptide dimerized by sulfhydryl group of cystein residue in the above described amino acid sequences.

9. An oligopeptide or a salt thereof represented by any one of the amino acid sequences (I) to (IX) described in claim 1.

10. A medicament comprising the oligopeptide claimed in any one of claim 1 to 8 or its physiologically acceptable salt as an active ingredient.

11. The medicament of claim 10, wherein the medicament is a hair growth promoting agent.

12. A use of the oligopeptide claimed in any one of claim 1 to 8 or its physiologically acceptable salt for manufacture of the medicament of claim 10 or 11.

13. A method for promoting hair growth, which comprises a step of administering an effective amount of the oligopeptide claimed in any one of claim 1 to 8 or its pharmaceutically acceptable salt to a mammal including human.


SEQUENCE LISTING

<110> Sumitomo Electric Industries, Ltd.

<120> Oligopeptide

<130> A11151M

<160> 9

<210> 1

<211> 11

<212> PRT

<213> human

<400> 1

Lys Ser Ile Glu Gln Ser Cys Asp Gln Asp Glu 11

<210> 2

<211> 10

<212> PRT

<213> human

<400> 2

Ser Ile Glu Gln Ser Cys Asp Gln Asp Glu 10

<210> 3

<211> 9

<212> PRT

<213> human

<400> 3

Ser Ile Glu Gln Ser Cys Asp Gln Asp 9

<210> 4

<211> 8

<212> PRT

<213> human

<400> 4

Ser Ile Glu Gln Ser Cys Asp Gln 8

<210> 5

<211> 7

<212> PRT

<213> human

<400> 5

Ser Ile Glu Gln Ser Cys Asp 7

<210> 6

<211> 6

<212> PRT

<213> human

<400> 6

Ser Ile Glu Gln Ser Cys 6

<210> 7

<211> 9

<212> PRT

<213> human

<400> 7

Ile Glu Gln Ser Cys Asp Gln Asp Glu 9

<210> 8

<211> 8

<212> PRT

<213> human

<400> 8

Glu Gln Ser Cys Asp Gln Asp Glu 8

<210> 9

<211> 7

<212> PRT

<213> human

<400> 9

Gln Ser Cys Asp Gln Asp Glu 7